# EUROPEAN PATENT SPECIFICATION

(11) **EP 2 061 404 B1**
(45) Date of publication and mention of the grant of the patent: **26.12.2012**
(21) Application number: 07796759.4
(22) Date of filing: 10.07.2007
(51) Int. Cl.: A61F 2/90, A61F 2/84

(54) **MULTILAYER SHEET STENT**
MEHRSCHICHTIGER FLÄCHENFÖRMIGER STENT
STENT A FEUILLES MULTICOUCHE

(30) Priority: 14.09.2006 US 844474 P; 26.06.2007 US 768304
(43) Date of publication of application: 27.05.2009
(73) Proprietor: Boston Scientific Limited, Christ Church (BB)
(72) Inventor: BROWN, Brian, Hanover, MN 55341 (US)
(74) Representative: Hauck Patent- und Rechtsanwälte
(86) International application number: PCT/US2007/015697
(87) International publication number: WO 2008/033177

(56) References cited:
- WO-A-98/22045
- US-A- 5 824 054
- US-A1- 2004 230 290
- US-A1- 2006 020 324

## Description

### BACKGROUND OF THE INVENTION

### Field of the Invention

In some embodiments this invention relates to implantable medical devices, their manufacture, and methods of use. Some embodiments are directed to delivery systems, such as catheter systems of all types, which are utilized in the delivery of such devices.

### Description of the Related Art

A stent is a medical device introduced to a body lumen and is well known in the art. Typically, a stent is implanted in a blood vessel at the site of a stenosis or aneurysm endoluminally, i.e. by so-called "minimally invasive techniques" in which the stent in a radially reduced configuration, optionally restrained in a radially compressed configuration by a sheath and/or catheter, is delivered by a stent delivery system or "introducer" to the site where it is required. The introducer may enter the body from an access location outside the body, such as through the patient's skin, or by a "cut down" technique in which the entry blood vessel is exposed by minor surgical means.

Stents, grafts, stent-grafts, vena cava filters, expandable frameworks, and similar implantable medical devices, collectively referred to hereinafter as stents, are radially expandable endoprostheses which are typically intravascular implants capable of being implanted transluminally and enlarged radially after being introduced percutaneously. Stents may be implanted in a variety of body lumens or vessels such as within the vascular system, urinary tracts, bile ducts, fallopian tubes, coronary vessels, secondary vessels, etc. They may be self-expanding, expanded by an internal radial force, such as when mounted on a balloon, or a combination of self-expanding and balloon expandable (hybrid expandable).

Stents may be created by methods including cutting or etching a design from a tubular stock, from a flat sheet which is cut or etched and which is subsequently rolled or from one or more interwoven wires or braids.

US 5,824,054 discloses a coiled sheet graft stent made of an expandable coiled sheet portion which is wound in turns at least three times.

WO 98/22045 discloses a microporous tubular prosthesis having a rolled sheet which is wound in at least three turns.

The technical problem of the invention is to provide a rolled sheet multilayer stent in which upon expansion the friction between two adjacent layers of the stent is avoided.

The problem is solved by a rolled sheet multilayer stent according to claim 1.

Without limiting the scope of the invention a brief summary of some of the claimed embodiments of the invention is set forth below. Additional details of the summarized embodiments of the invention and/or additional embodiments of the invention may be found in the Detailed Description of the Invention below.

### BRIEF SUMMARY OF THE INVENTION

In at least one embodiment, the invention is directed to a stent formed of at least two sheets/layers of material. In at least one embodiment, the at least two layers of material forming the stent are offset from one another. In at least one embodiment, the at least two layers of material forming the stent can move relative to one another. In at least one embodiment, the at least two layers forming the stent has a tapered end. In at least one embodiment, one of the at least two layers has an open cell design and one of the at least two layers has a closed cell design and one layer is positioned on top of the other layer. In at least one embodiment, each of the at least two layers has a portion of the layer with an open cell design and a portion of the layer with a closed cell design so that when the layers are placed on top of one another the open cell design of the top layer is on the closed cell design of the bottom layer and the closed cell design of the top layer is on the open cell design of the bottom layer.

These and other embodiments which characterize the invention are pointed out with particularity in the claims annexed hereto and forming a part hereof. However, for further understanding of the invention, its advantages and objectives obtained by its use, reference can be made to the drawings which form a further part hereof and the accompanying descriptive matter, in which there is illustrated and described an embodiments of the invention.

### BRIEF DESCRIPTION OF THE SEVERAL VIEWS OF THE DRAWING(S)

A detailed description of the invention is hereafter described with specific reference being made to the drawings.
FIG. 1a is a flat view of a sheet with an example of a closed cell design.
FIG. Ibis a flat view of a sheet with an example of an open cell design.
FIG. 1c-1e are flat views of sheets each with an example of a free-form cell design.
FIG. 1f is a flat view of a sheet with multiple cell designs.
FIG. 2a is a flat view of the cell design of a multilayer rolled stent where the top layer is a closed cell design and the bottom layer is an open cell design.
FIG. 2b is a side view of the stent in FIG. 2a in an unrolled state.
FIG. 2c is an end view of the stent in Fig. 2a in an unrolled state.
FIG. 3 is a flat view of a multilayer rolled stent where the three layers of FIGs. 1c-1e are laid on top of one another.
FIG. 4a is a flat view of a multilayer rolled stent where both layers have an open cell stent design and the ends of the layers are off-set or de-registered.
FIG. 4b is longitudinal side view of the stent in Fig. 4a in an unrolled state.
FIG. 5a is a longitudinal side view of a multilayer rolled stent in an unrolled state with a taper.
FIG. 5b is a side view of the multilayer rolled stent showing the taper.
FIG. 6 is an end view of a multilayer rolled stent in an unrolled state where the edges of the layers are off-set or de-registered.
FIG. 7a is a cross-sectional view of a multilayer rolled stent where the circumferential length of the layer is less than the circumferential length of the stent.
FIG. 7b is a cross-sectional view of a multilayer rolled stent where the first edge of the first layer overlaps the second edge of the first layer and the first edge of the second layer overlaps the second edge of the second layer.
FIG. 7c is a cross-sectional view of a multilayer rolled stent where the second edges of the layers overlap the first edges of the layers.
FIG. 7d is a cross sectional view of a multilayer rolled stent where the layers are tapered at the edges and the second edges of the layers overlap the first edges of the layers.
FIG. 7e is a cross sectional view of the stent in Fig. 6d with the layers having the same circumferential length.
FIG. 7f is a cross sectional view of a multilayer rolled stent of Fig. 6 in a rolled state.
FIG. 7g is a cross sectional view of a multilayer rolled stent where the edges of the layers are circumferentially offset from one another.
FIG. 8a is a side view of a catheter assembly with the inventive stent.
FIG. 8b is a side view of another catheter assembly with the inventive stent.
FIG. 8c is a side view of a third catheter assembly with the inventive stent.

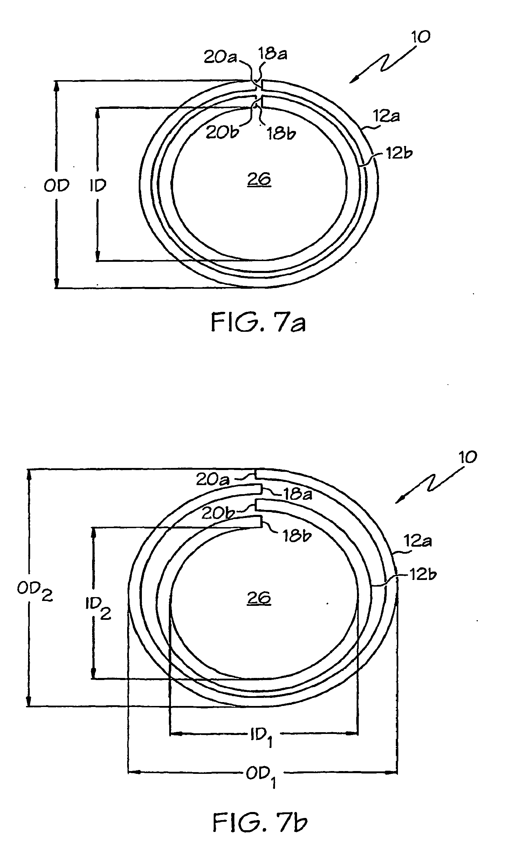

FIG. 7a shows an embodiment of the invention, while FIGS. 7b to 7g show further examples of multilayer rolled not according to the invention.

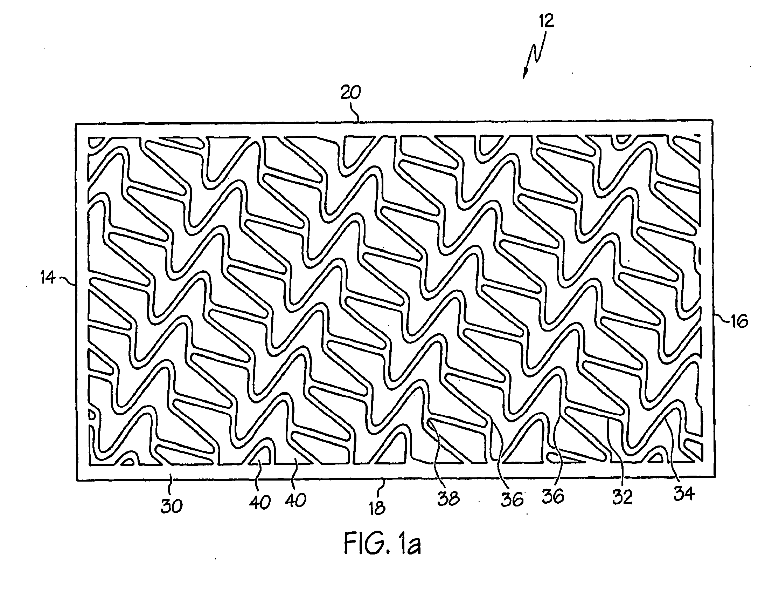

Figures 1a-f, 2a-c, 4a,b and 7a-e disclose embodiments of the invention.

### DETAILED DESCRIPTION OF THE INVENTION

While this invention may be embodied in many different forms, there are described in detail herein specific embodiments of the invention. This description is an exemplification of the principles of the invention and is not intended to limit the invention to the particular embodiments illustrated.

For the purposes of this disclosure, like reference numerals in the figures shall refer to like features unless otherwise indicated.

The invention is directed to a multilayer rolled stent 10 formed from a plurality of sheets/layers 12 of material. The use of multiple layers 12 to form the stent 10 can decrease the overall cell size of the stent 10. The smaller cells 40 of a multilayer rolled stent 10 reduce the likelihood that embolic particles are shed from the implant site. In addition, the scaffolding properties of the original cell design are multiplied in a multilayer rolled stent 10.

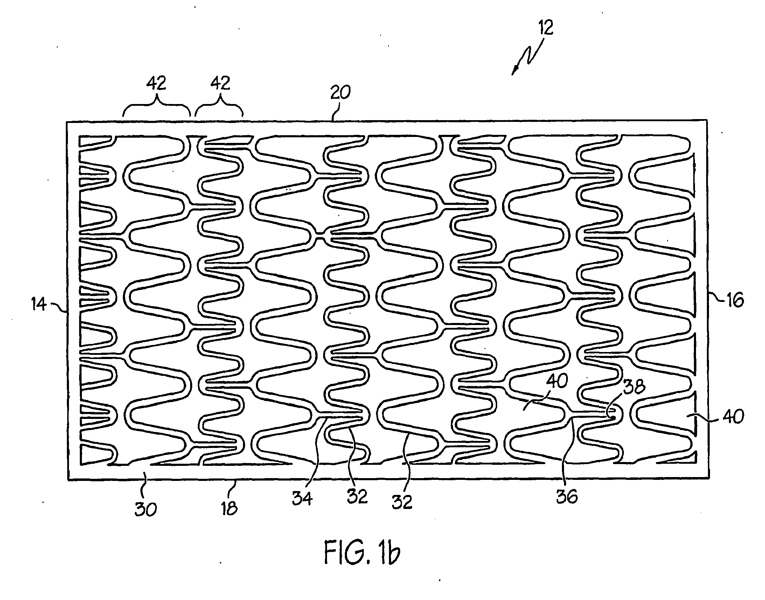

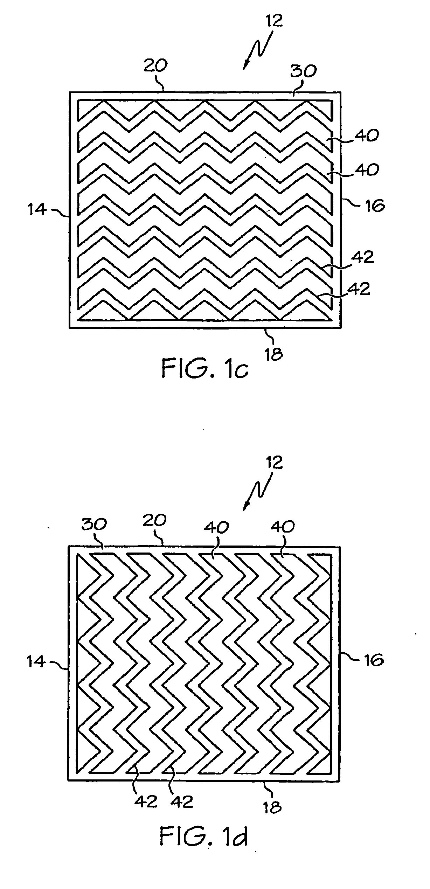

Figures 1a, 1b, and 1c-e illustrate examples of cell designs that can be used for the individual layers 12 of the multilayer rolled stent 10. Each layer 12 has a longitudinal length, a circumferential length, a proximal end 14, a distal end 16, a first edge 18 and a second edge 20. The longitudinal lengths of each layer 12 of the multilayer rolled stent 10 may be the same length or different lengths. In at least one embodiment, each layer 12 forming the multilayer rolled stent 10 has the same longitudinal length. In at least one embodiment, each layer 12 forming the multilayer rolled stent 10 may or may not have the same longitudinal length. In at least one embodiment, each layer 12 forming the multilayer rolled stent 10 has a different longitudinal length. In at least one embodiment, each layer 12 forming the multilayer rolled stent 10 has the same circumferential length. In at least one embodiment, each layer 12 forming the multilayer rolled stent 10 may or may not have the same circumferential length. In at least one embodiment, each layer 12 forming the multilayer rolled stent 10 has a different circumferential length.

Each layer 12 will have a band of material without cells 40 around the perimeter of the layer 12 with the stent design within this perimeter band 30. Figure 1a is an example of a closed cell stent design. A closed cell design is characterized by a connector 34 extending between every turn 36 of a circumferential band 42.

In contrast, an open cell design is characterized by a connector 34 extending at most between every other turn 36 of a circumferential strut 32 and at the least between only one turn 36 of a circumferential band 42 and one turn 36 of an adjacent circumferential band 42. Open cell designs usually provide better longitudinal flexibility than closed cell designs.

Connectors 34 can extend from a turn 36 on one circumferential band 42 to a valley 38 on the adjacent circumferential band 42, as shown in Fig. 1b or extend from a turn 36 on one circumferential band 42 to a turn 36 on the adjacent circumferential band 42, as shown in Fig. 1a. Open and closed cell designs may be perpendicular to the longitudinal axis of the stent 10, as shown in Fig. 1b or may be at an oblique angle to the longitudinal axis, as shown in Fig. 1a. Unless otherwise noted, an oblique angle as used in this application is an angle between 0-180 degrees and includes 90 degrees.

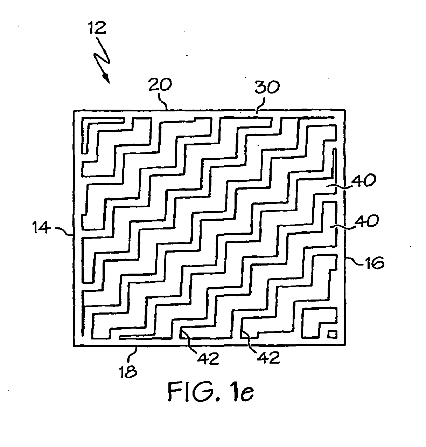

Examples of free-form cell designs are illustrated in Figs. 1c-e. A free-form cell design consists of a plurality of struts 32. In this example the plurality of struts 32 form a plurality of zig-zag bands 42. The zig-zag bands 42 forming the free-form designs of the layers 12 are oriented in different angles, e.g. longitudinal orientation (Fig. 1c), circumferential orientation (Fig. 1d) and at an oblique angle to the longitudinal axis (Fig. 1e). A free-form cell design does not have any connectors 34 engaging adjacent bands 42 such as is seen in the closed cell design and the open cell design.

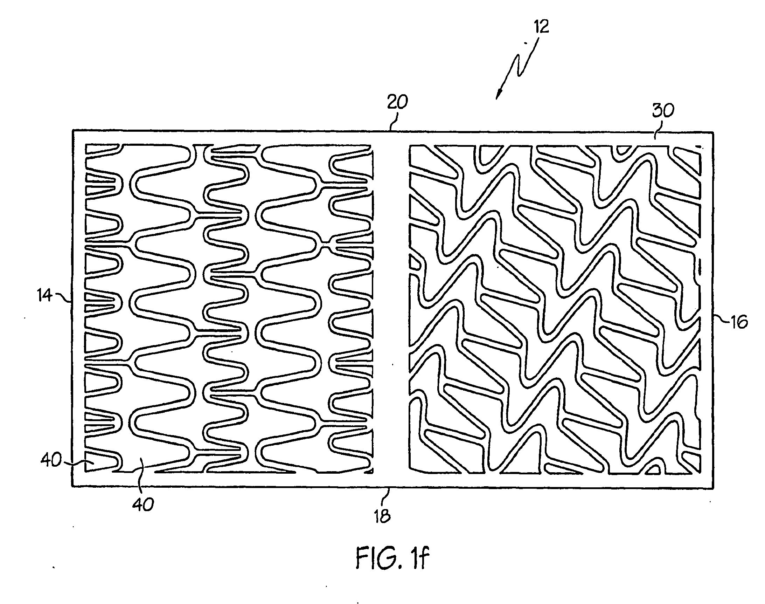

Figure 1f illustrates that a single layer 12 can have a plurality of cell designs. In the embodiment shown, there are two cell design sections along the longitudinal length of the layer 12, an open cell design section and a closed cell design section. The two design sections are separated by a band of stent material. In this embodiment, the band of stent material is a circumferential band. In at least one embodiment, the two design sections are separated by a longitudinal band of stent material. It will be appreciated that there can be any number of design sections along the longitudinal length of the layer 12, depending upon the size of the design sections and the size, i.e. length and width, of the layer 12. Thus, it is within the scope of the invention for a layer 12 to have one, two, three, four, five, six, seven, eight, nine, ten or more design sections. It is also within the scope of the invention for the layer 12 to have at least four design sections separated by a longitudinal band and a circumferential band.

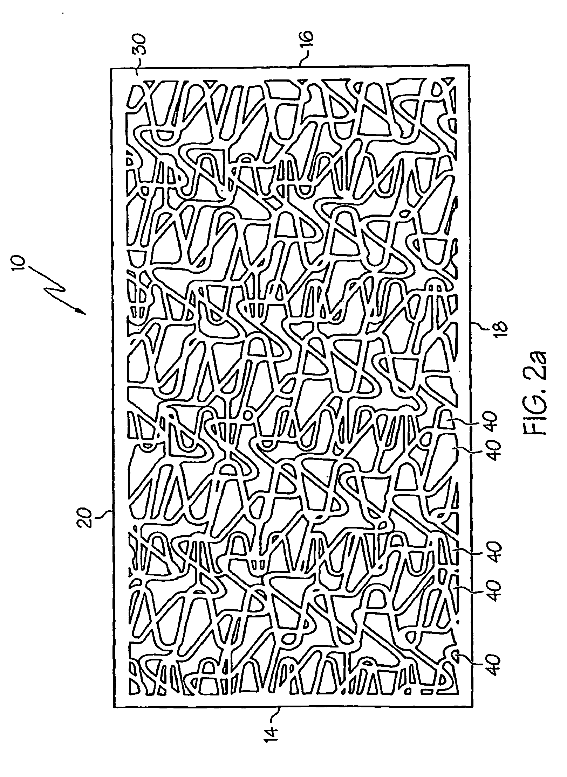

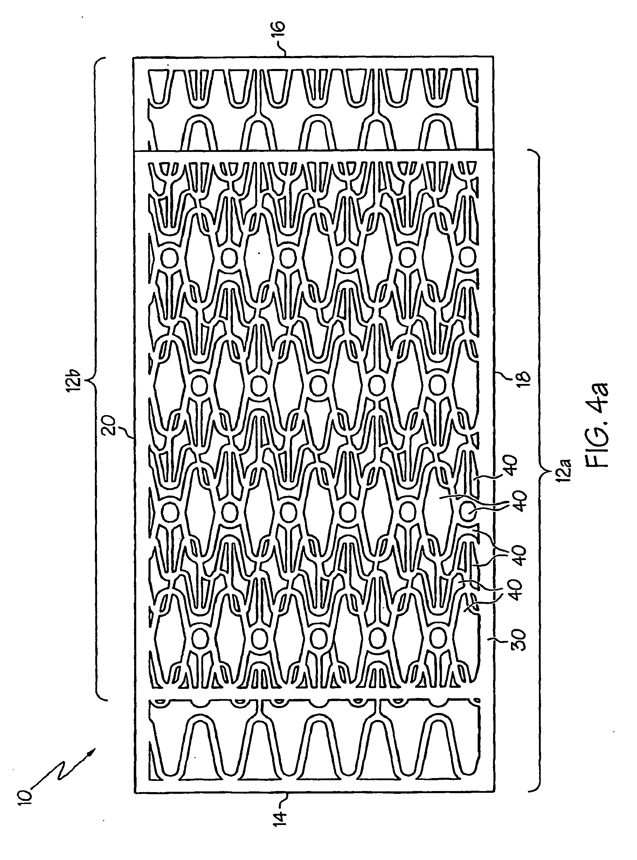

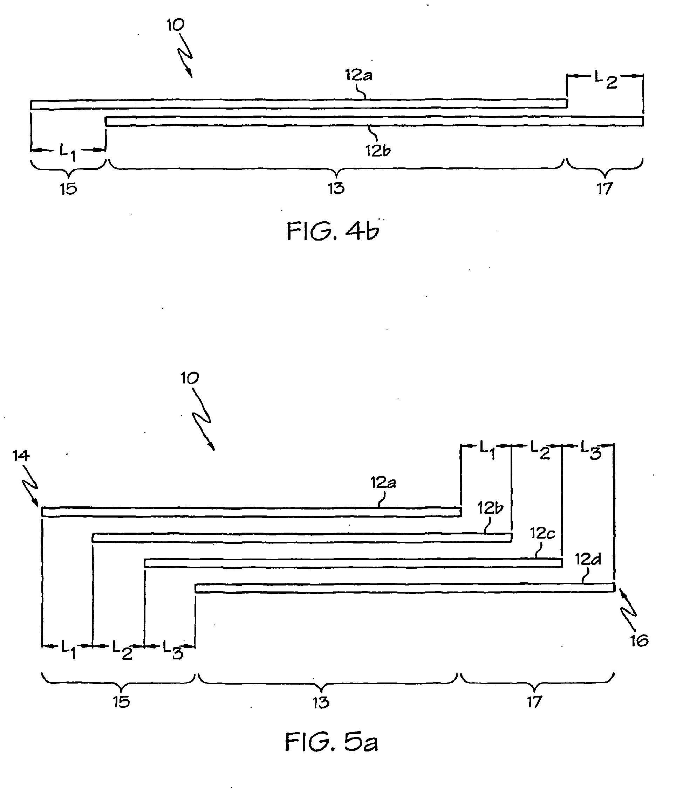

All the embodiments of the multilayer rolled stent 10 described herein have an expanded rolled state, an unexpanded rolled state and an unrolled state. The multilayer rolled stent 10 is in an expanded rolled state when deployed within a body lumen while the multilayer rolled stent 10 is in an unexpanded rolled state when engaged to the delivery system. When the multilayer rolled stent 10 is in either an expanded or unexpanded rolled state, one of the layers 12 forms at least a substantial portion of the outer surface layer of the multilayer rolled stent 10 and another layer 12 forms a substantial portion of the inner surface layer of the multilayer rolled stent 10, as illustrated in Fig. 4a and b for example. The inner surface layer defines the flow path 26 of the multilayer rolled stent 10. In at least one embodiment, one layer 12 forms the entire outer surface layer of the multilayer rolled stent 10 and a second layer 12 forms the entire inner surface layer of the multilayer rolled stent 10, as illustrated in Figs. 2a-c, for example.

All of the embodiments herein must have a minimum of two layers 12 but otherwise the multilayer rolled stent 10 may be constructed with a different number of layers 12 than illustrated for the particular embodiment. In at least one embodiment, the multilayer rolled stent 10 has at least two layers 12. Thus it is within the scope of the invention for the multilayer rolled stent 10 to have two, three, four, five, six, seven, eight or more layers 12.

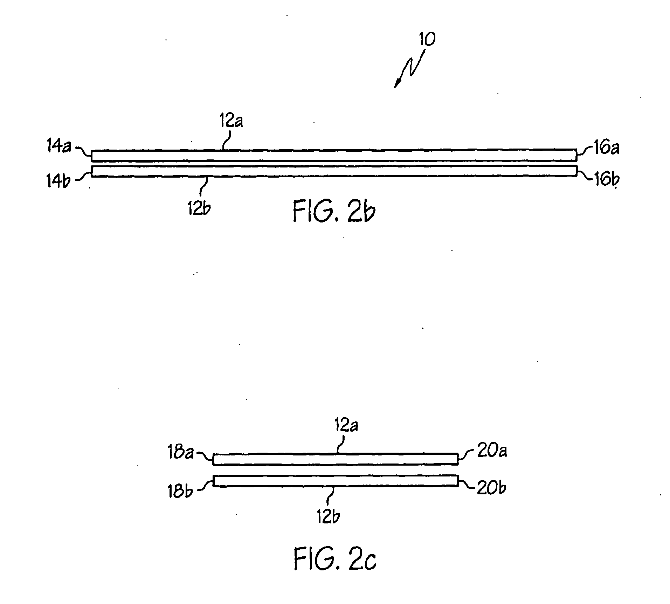

In the embodiment shown in Fig. 2a and 2b, the multilayer rolled stent 10 has two layers 12a,b, as shown in the longitudinal side view of the unrolled multilayer rolled stent 10 in Fig. 2b and the end view of the unrolled multilayer rolled stent 10 in Fig. 2c. As illustrated in Fig. 2b and 2c, the proximal ends 14a,b, and the distal ends 16a,b of the layers 12a,b are aligned and together form the proximal end 14 and the distal end 16 of the multilayer rolled stent 10. In addition, the first edges 18a,b and the second edges 20a,b are aligned and together form the first edge 18 and second edge 20 of the multilayer rolled stent 10. In all of the side view illustrations of the multilayer rolled stent 10 in an unrolled state and the end view in Fig. 2c, the layers 12 were drawn with spaces between them in order to clearly show the different layers 12 comprising the multilayer rolled stent 10. In actuality, the different layers 12 of the multilayer rolled stent 10 would be laying on top of one another similar to sheets of paper laid on top of one another.

In this embodiment, the top layer 12a has the closed cell design of Fig. 1a and the bottom layer 12b has the open cell design of Fig. 1 b. The cells 40 that result from this overlay are shown in Fig. 2a. Note, that as shown in Fig. 2a, the multilayer rolled stent 10 has cells 40 which are smaller than the cells 40 of the individual layers 12a, b.

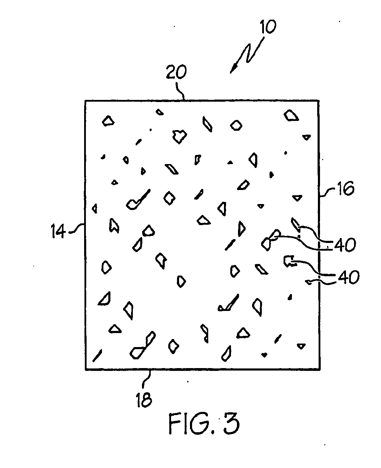

In Fig. 3, the multilayer rolled stent 10 has three layers 12a,b,c with the designs of Figs. 1c-e which results in the cells 40 shown. The size of the cells 40 on each layer 12 affects the size of the cells 40 of the multilayer rolled stent 10. If the individual layers 12 have large size cells 40, then the cells 40 formed when the layers 12 are placed together will be larger than if the individual layers 12 have smaller size cells 40, as can be seen when the size of the cells 40 of the individual layers 12 in Figs. 1c-e are compared to the size of the cells 40 of the mulitlayer rolled stent 10, shown in Fig. 3. Thus, the designs of the individual layers 12 can be chosen so that the cells 40 of the multilayer rolled stent 10 are the desired size.

Figures 4a and b show an alternative embodiment of the multilayer rolled stent 10. In this embodiment the layers 12a, b of the multilayer rolled stent 10 are offset or de-registered from one another in a longitudinal direction so that the total longitudinal length of the multilayer rolled stent 10 is greater than the longitudinal length of the individual layers 12a, b. This can be seen in Fig. 4b which is a side view of the unrolled multilayer stent 10. The multilayer rolled stent 10 also has a proximal end region 15 which has a length L1, which is the length of the offset, a distal end region 17, which has a length L2, which is the length of the offset and a middle region 13 which is between the proximal end region 15 and the distal end region 17. In this embodiment, the proximal end region is the same length as the distal end region. In at least one embodiment, the length of the proximal end region is different than the length of the distal end region.

In the embodiment shown in Figs. 4a and 4b, both the top layer 12a and the bottom layer 12b have the open cell design shown in Fig. 1b. Note that the cells 40 formed, by placing one layer 12a on the second layer 12b, are smaller than the cells 40 of the individual layers 12a, b. Also note that the multilayer rolled stent 10 will have a slight taper at both the proximal end region 15 and the distal end region 17 because at the end regions 15,17 there is only one layer 12 due to the longitudinal off-setting or deregistering of the two layers 12a,b from one another. Although not illustrated, the first edges 18 of the layers 12a,b are aligned to form the first edge 18 of the multilayer rolled stent 10 in an unrolled state, similar to Fig. 2c. The layers 12 of the multilayer rolled stent 10 are off-set from one another in a circumferential direction so that the first edges 18 and the second edges 20 of the layers 12 are not aligned.

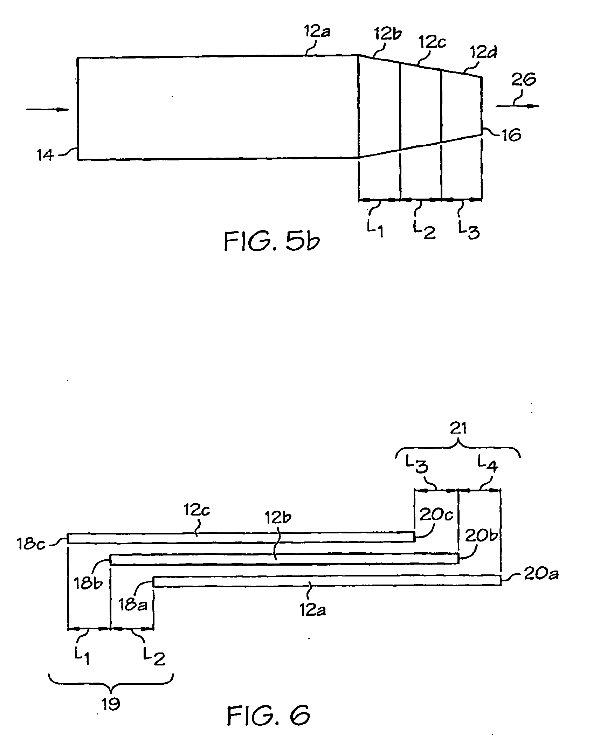

In the embodiment shown in Fig. 5a and 5b, the multilayer rolled stent 10 has four layers 12a,b,c and d that are offset from one another in a longitudinal direction. The multilayer rolled stent 10 also has a proximal end region 15 which has a length L1+L2+L3, which is the length of the offset, a distal end region 17, which has a length L1+L2+L3, which is the length of the offset and a middle region 13 which is between the proximal end region 15 and the distal end region 17. In this embodiment, the proximal end region 15 the same length as the distal end region 17. In at least one embodiment, the length of the proximal end region 15 is different than the length of the distal end region 17. As illustrated in the longitudinal cross-section of Fig. 5a, the proximal end 14 of the first layer 12a is the proximal end 14 of the multilayer rolled stent 10 while the distal end 16 of the fourth layer 12d is the distal end 16 of the multilayer rolled stent 10. In this embodiment, each layer 12 is offset from the next layer 12 by an equal length L. Thus, layer 12a is offset from layer 12b by length L1, layer 12b is offset from layer 12c by length L2, layer 12c is offset from layer 12d by length L3 and L1=L2=L3. However, in at least one embodiment the amount of offset between layers 12 is different, i.e. lengths L1≠L2≠L3. Thus, it is apparent that there are many possible combinations of offsets between the layers 12 and it is within the scope of the invention for the off-set between layers 12 to have any length.

In addition, in this embodiment the lengths L1, L2 and L3 are the same on both the proximal end region 15 and the distal end region 17 because each layer 12 has the same longitudinal length. However, the layers 12 could have different longitudinal lengths so that the lengths of the overlaps at the proximal end region 15 are different from the lengths of the overlaps at the distal end region 17.

Figure 5b shows the multilayer rolled stent 10 of Fig. 5a in a rolled form. The multilayer rolled stent 10 is a substantially circular tube with a tapered inner diameter at the proximal end region 15 and a tapered outer diameter at the distal end region 17. The inner diameter would be largest at the proximal end region 15 and smallest at the distal end region 17. Similarly, the outer diameter would be largest at the proximal end region 15 and smallest at the distal end region 17. The tapered inner diameter results in a tapered flow path 26. Figures 7a and 7b show how the inner diameter and the outer diameter can be measured for a rolled stent 10.

The change in the inner and outer diameters over the taper depends upon the thickness of the layers 12 in both the tapered proximal and distal end regions 15,17 and in the non-tapered middle region 13. If the layers 12 have a thick non-tapered middle region 13 and thin tapered proximal and distal end regions 15,17, then the taper is more pronounced, i.e. the change in the diameters is great. However, if the layers 12 have a constant thickness throughout and the thickness is quite small, then the taper is minimal, i.e. the change in the diameters is small. In addition, the length of the taper is determined by the lengths of the offsets L1, L2, etc which comprise the proximal and distal end regions 15,17. Thus, in this embodiment, the length of the taper is equal to the sum of L1, L2, and L3. However, if the layers 12 have different longitudinal lengths then the length of the taper at the proximal end region 15 of the multilayer rolled stent 10 can be different than the length of the taper at the distal end region 17. Thus, the tapered inner diameter, flow path 26, would be longer than the tapered outer diameter.

To achieve the tapered rolled multilayer rolled stent 10 shown in Fig. 5b, the layers 12 shown in Fig. 5a are rolled from the first edge 18 to the second edge 20 at a right angle to the first edge 18 to form a substantially tubular multilayer rolled stent 10. Another method to produce a tapered rolled multilayer rolled stent 10 is to roll layers that are not offset, as shown in Fig. 2b. In this method the layers 12 are rolled from the first edge 18 to the second edge 20 at an oblique angle to the first edge 18 thereby forming a substantially tubular multilayer rolled stent 10 with a taper. An oblique angle in this instance would be between one and eighty-nine degrees.

In at least one example, the first edges 18 and the second edges 20 of the layers 12a,b,c are off-set or de-registered, as illustrated by the multilayer rolled stent 10 of Fig. 6, which is in an unrolled state. Thus, the edges 18,20 of the layers 12 do not form the first edge 18 and second edge 20 of the multilayer rolled stent 10. In this embodiment, the first edges 18a,b of the first and second layers 12a,b are offset by a length equal to L2 and the first edges 18b,c of the second and third layers 12b,c are offset by a length equal to L1. Similarly, the second edges 20a,b of the first and second layers 12a,b are offset by a length equal to L3 and the second edges 20b,c of the second and third layers 12b,c are offset by a length equal to L1. Thus the multilayer rolled stent 10 has a first edge region 19 which has a length equal to L1+L2 and a second edge region 21 which has a length equal to L3+L4. It is within the scope of the invention for the off-set between layers 12 to be any length. In one embodiment, L1=L3 and L2=L4. In one example L1, L2, L3 and L4 are different lengths. In one example, the length of the first edge region 19 is equal to the length of the second edge region 21.

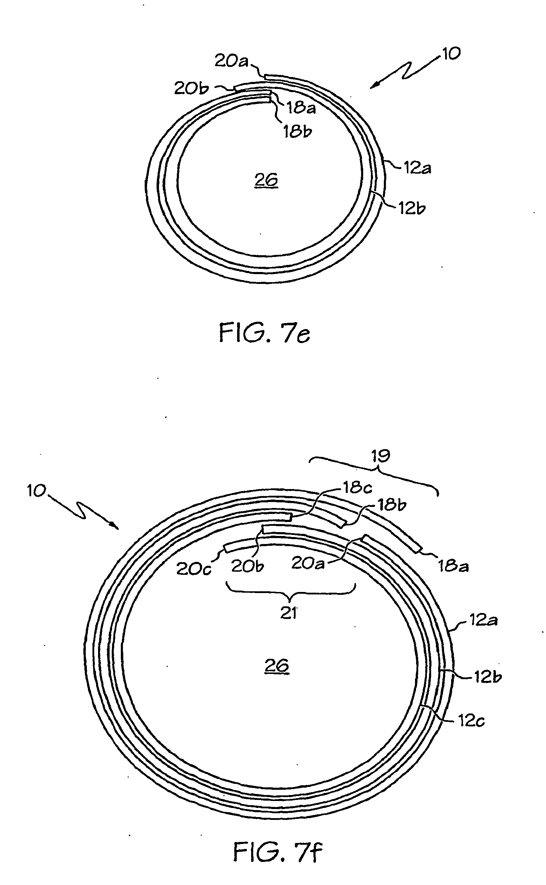

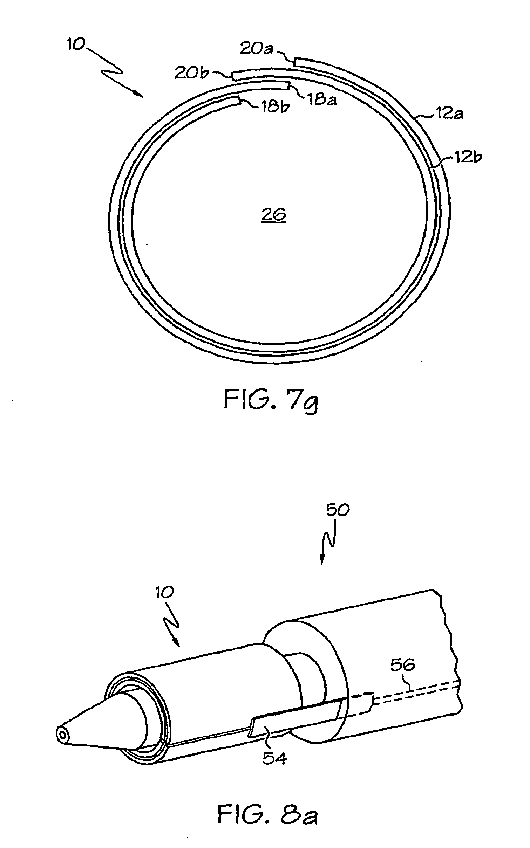

Figures 7a-g are cross-sectional views of the inventive multilayer rolled stent 10 showing the various ways the layers 12a,b could be arranged in a rolled state to form the substantially tubular form of the multilayer rolled stent 10. These different ways to form the substantially tubular form of the multilayer rolled stent 10 can be used for any of the multilayer rolled stent 10 embodiments described herein. Note that although Figs7a-g illustrate the multilayer rolled stent 10 being rolled, for example, from the first edge towards the second edge, the stent 10 can also be rolled in the opposite direction. These opposite ways of forming the multilayer rolled stent 10 are illustrated, for example, in Figs. 7f and 7g. An embodiment of the present invention is shown in Fig. 7a. Each layer 12a,b forms an incomplete circle, so that there is a small space between the first edge 18a of the first layer 12a and the second edge 20a of the first layer 12a. Thus, the first edge 18a of the first layer 12a is opposite the second edge 20a of the first layer 12a. Similarly, the first edge 18b of the second layer 12b is opposite the second edge 20b of the second layer 12b. In at least one embodiment, the distance between the first edge 18a and the second edge 20a of the first layer 12a is equal to zero, so that there is no gap or space between the edges 18,20. Thus, it is within the scope of the invention for the gap between the edges 18,20 to be 0 mm to 0.5 mm. In the embodiment illustrated in Fig. 7a, both layers 12a,b have the same thickness, however, it is within the scope of the invention for the layers 12 to have different thicknesses. Also, in this embodiment, the first layer 12a has a slightly greater circumferential length than the inner layer 12b. The innermost layer 12, which in this embodiment is layer 12b, defines a flow path 26 for the multilayer rolled stent 10. The outermost layer 12, which in this embodiment is layer 12a, forms the outer surface layer 12 of the multilayer rolled stent 10.

Note that there is also a slight space between the first layer 12a and the second layer 12b. This allows the first layer 12a to move relative to the second layer 12b in either an axial or longitudinal direction. In at least one embodiment, the layers 12 of the multilayer rolled stent 10 do not move relative to one another.

For every multilayer rolled stent 10 embodiment described herein using the means of arranging the layers 12 of the multilayer rolled stent 10 into a substantially tubular body as illustrated in Fig. 7a, the outermost layer 12 will have the longest circumferential length while the innermost layer 12 will have the shortest circumferential length and the circumferential lengths of any layers 12 in between the outer surface layer 12 and the inner surface layer 12 will be progressively shorter the closer the layer 12 is to the inner surface layer 12.

In Fig. 7b, the second edge 20a of the first layer 12a overlaps the first edge 18a of the first layer 12a. Similarly, the second edge 20b of the second layer 12b overlaps the first edge 18b of the second layer 12b. The second layer 12b again defines the flow path 26 for the multilayer rolled stent 10. Note that in this embodiment, the space between the two layers 12 is greater than that needed for the embodiment of Fig. 7a due to the overlapping of the second edges 20 over the first edges 18. Note that if the overlap of the edges is the same for every layer 12, the outermost layer 12a will have the longest circumferential length and the innermost layer 12b will have the shortest circumferential length. If the outermost layer 12a and the innermost layer 12b have the same circumferential length then the overlap of the second edge 20 over the first edge 18 would be greater for the innermost layer 12b than for the outermost layer 12a.

The multilayer rolled stent 10 has an outer diameter OD and an inner diameter ID, as shown for example in Figs. 7a and 7b. The inner diameter ID is measured from opposite points on the inner surface of the innermost layer 12, which would be the inner surface layer 12 of the multilayer rolled stent 10. The outer diameter OD is measured from opposite points on the outer surface of the outermost layer 12, which would be the outer surface layer 12 of the multilayer rolled stent 10. At any point about the circumference of the multilayer rolled stent 10, the thickness of the stent 10 at a particular position can be ascertained by subtracting the inner diameter ID from the outer diameter OD (OD-ID).

The thickness (OD-ID) of the multilayer rolled stent 10 at different positions about the circumference of the stent 10 can either be substantially constant or can vary. In Fig. 7a, the thickness (OD-ID) of the body of the multilayer rolled stent 10, is substantially constant. In Fig. 7b, the thickness of the body of the multilayer rolled stent 10 varies about the circumference of the multilayer rolled stent 10. As shown in Fig. 7b, OD1-ID1 is different from OD2-ID2. This difference is due to the overlapping edges 18,20.

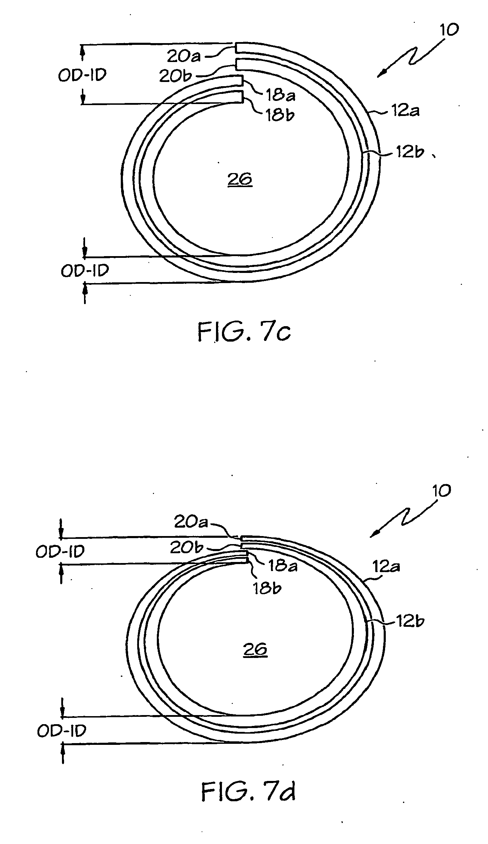

Figures 7c-e illustrate different embodiments of the same concept. In these embodiments, the second edges 20a,b of both the first layer 12a and the second layer 12b overlap the first edges 18a,b of both the first layer 12a and the second layer 12b. Again, the first layer 12a is the outer surface layer 12 of the multilayer rolled stent 10 and the second layer 12b is the inner surface layer 12 and defines a flow path 26 for the multilayer rolled stent 10. Unlike the embodiment of Fig. 7b, the space between the two layers 12 can remain as small as possible yet allow the two layers 12 to move relative to one another. Because the two layers 12 have the same thickness, the tubular shape of the multilayer rolled stent 10 in Fig. 7c is more oval than circular due to the overlapping edges 18,20.

In Fig. 7d, the tubular shape of the multilayer rolled stent 10 in Fig. 7d is more circular because the thickness of the two layers 12 in the first end region and the second end region tapers toward the edges 18, 20. The length of the taper can vary from 0.5 mm to 5 mm, or the entire diameter of the multilayer rolled stent 10 in an expanded state. Thus, the edges 18,20 are thinner than middle portion of the layer 12. Note that the first and second end regions of the cross-section of Fig. 7b can also be tapered similar to the tapered ends of Fig. 7d. In at least one embodiment, tapering the first and second end regions of the layers 12 in Fig. 7b decreases the amount of space between the two layers 12. When the first and second end regions of the layers 12 are tapered, the variation in the thickness (OD-ID) of the body of the multilayer rolled stent 10 is minimized, as can be seen by comparing the thickness (OD-ID) at two different positions about the circumference of the multilayer rolled stents 10 in Figs. 7c and 7d.

Also note that there can be a greater overlap of the second edge 20 over the first edge 18 than is shown in the embodiments illustrated in Fig. 7b, 7c and 7d. Factors that influence the amount of overlap include, but are not limited to, the circumferential length of the layers 12, the circumference of the multilayer rolled stent 10 in the rolled state and whether the multilayer rolled stent 10 is in an unexpanded state or in an expanded state. In at least one embodiment, the overlap of the layers 12 is substantially the same when the multilayer rolled stent 10 is in an unexpanded state and in an expanded state. In at least one embodiment, the overlap of the layers 12 changes when the multilayer rolled stent 10 goes from an unexpanded state to an expanded state.

In Fig. 7e, the innermost layer 12b and outermost layer 12a of the multilayer rolled stent 10 have the same circumferential length. Because the layers 12 have the same circumferential lengths, the second edge 20 of the inner layer 12b overlaps the first edges 18 of the layers 12 to a greater extent than the second edge 20 of the outer layer 12a. Any layers 12 between the outermost layer 12a and the innermost layer 12b will overlap the first edges 18 to varying degrees, with the innermost layer 12b having the greatest overlap and the outermost layer 12a having the smallest amount of overlap. Note that in Fig. 7e the edges 18,20 of the multilayer rolled stent 10 are tapered but the edges may 18,20 not to have a taper.

Figure 7f illustrates the multilayer rolled stent 10 of Fig. 6, with off-set edges 18,20 in a rolled state. In the rolled state, the layers 12a,b,c of the multilayer rolled stent 10 are circumferentially offset. The innermost layer 12c defines a flow path 26 for the multilayer rolled stent 10. The outermost layer 12a forms the outer surface layer 12 of the multilayer rolled stent 10. In the rolled state, the first edge 18,b,c of each layer 12a,b,c overlaps the second edge 20a,b,c of each layer 12a,b,c so that the first edge 18c of the third layer 12c overlaps the second edge 20c of the third layer 12c, the first edge 18b of the second layer 12b overlaps the second edge 20b of the second layer 12b and the first edge 18a of the first layer 12a overlaps the second edge 20a of the first layer 12a. In essence, the first edge region 19 overlaps the second edge region 21 of the multilayer rolled stent 10. In one embodiment, the first edge region 19 overlaps at least a portion of the second edge region 21. In at least one example, the layers 12 are the same size. In at least one example, the layers 12 have different circumferential lengths. In at least one example, the edges 18,20 of the layers 12 are off-set and the ends 14,16 are offset.

In Fig. 7g, the innermost layer 12b and the outermost layer 12a of the multilayer rolled stent 10 have the same circumferential length but the edges 18a,b and 20a,b of each layer 12a,b are offset from one another. As shown in Fig. 7g, in the rolled state, the layers 12a,b,c of the multilayer rolled stent 10 are circumferentially offset. Thus, the first edge 18a of the first layer 12a overlaps the first edge 18b of the second layer 12b and the second edge 20b of the second layer 12b overlaps the first edge 18a of the first layer 12a. However, the second edge 20b of the second layer 12b does not overlap the first edge 18b of the second layer 12b and similarly, the second edge 20a of the first layer 12a does not overlap the first edge 18a of the first layer 12a. In at least one embodiment, the layers 12 are the same size. In at least one example, the layers 12a,b do not have the same circumferential length and the edges 18a,b and 20a,b of each layer 12a,b are offset from one another. In at least one example, the edges 18,20 of the layers 12 are off-set and the ends 14,16 are offset.

The multilayer rolled stent 10 can be delivered via a catheter assembly 50 as shown in Figs. 8a-c. In Fig. 8a, the multilayer rolled stent 10 is engaged to the catheter assembly 50 by a restraining clip 54. When the catheter assembly 50 is in the proper place within the vasculature, the restraining clip 54 is pulled backed by a pull wire 56 to allow the multilayer rolled stent 10 to assume an expanded state in the body lumen, typically a vessel. The pull wire 56 extends along the length of the catheter assembly 50 to the proximal end of the catheter assembly 50.

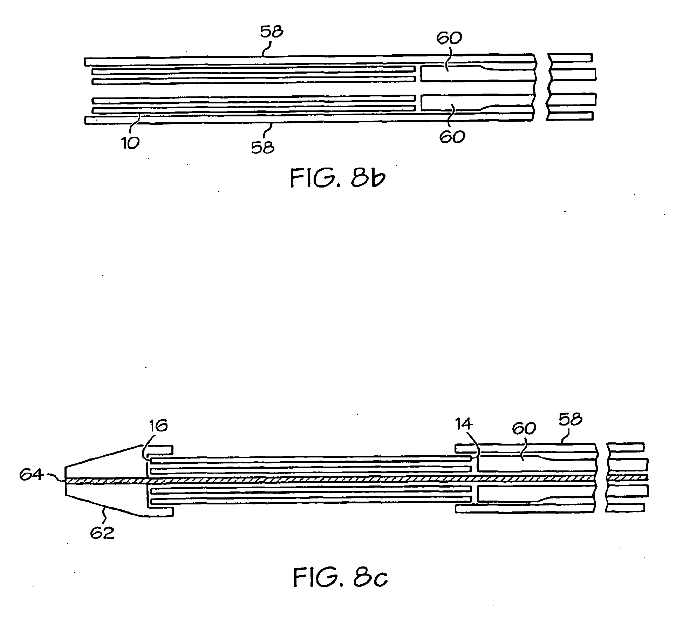

Figure 8b shows another catheter assembly 50 that can be used to deliver a multilayer rolled stent 10. The catheter sheath 58 keeps the multilayer rolled stent 10 in an unexpanded state while the catheter assembly 50 is advanced through the vasculature. When the catheter assembly 50 reaches the site where the multilayer rolled stent 10 is to be deployed, the push rod 60 can be moved distally to push the multilayer rolled stent 10 out from under the catheter sheath 58. Alternatively, the push rod 60 can keep the multilayer rolled stent 10 in position while the catheter sheath 58 is withdrawn and uncovers the multilayer rolled stent 10. Once the catheter sheath 58 is withdrawn, the multilayer rolled stent 10 will assume an expanded state within the body lumen, typically a vessel.

Figure 8c is an example of the catheter assembly 50 of Fig. 8b where the catheter assembly 50 has a distal stent retainer 62 as well as a push rod 60 and a catheter sheath 58. In this embodiment, the catheter sheath does not cover the entire longitudinal length of the multilayer rolled stent 10 but only covers the proximal end 14 of the multilayer rolled stent 10. The distal end 16 of the multilayer rolled stent 10 is held in place by the distal stent retainer 62, which is controlled by a release rod 64 that extend to the proximal end of the catheter assembly 50. Once the catheter assembly 50 is in the desired location the multilayer rolled stent 10 may be released from the catheter assembly 50 in one of two ways. The first method is to push the release rod 64 in a distal direction so that the distal stent retainer 62 releases the distal end 16 of the multilayer rolled stent 10 and the withdrawing the catheter sheath 58 to release the proximal end 14 of the multilayer rolled stent 10. The second method is to withdraw the catheter sheath 58 to release the proximal end 14 of the multilayer rolled stent 10 and then the push the release rod 64 in a distal direction so that the distal stent retainer 62 releases the distal end 16 of the multilayer rolled stent 10. After the multilayer rolled stent 10 is released from the catheter assembly 50, it will assume an expanded state within the body lumen, typically a vessel.

It will be appreciated that Figs. 8a, b and c illustrate only a few means by which the multilayer rolled stent 10 may be retained onto the catheter assembly 50. Examples of other means to retain the multilayer rolled stent 10 onto the catheter assembly 50 include, but are not limited to, rings, pull-strings, string wraps, bars, and a catheter sleeve and electrolytic fusible joint of fusible link.

The sheets forming the layers 12 of the multilayer rolled stent 10 may be made from any suitable biocompatible materials including one or more polymers, one or more metals or combinations of polymer(s) and metal(s). Examples of suitable materials include biodegradable materials that are also biocompatible. By biodegradable is meant that a material will undergo breakdown or decomposition into harmless compounds as part of a normal biological process. Suitable biodegradable materials include polylactic acid, polyglycolic acid (PGA), collagen or other connective proteins or natural materials, polycaprolactone, hylauric acid, adhesive proteins, copolymers of these materials as well as composites and combinations thereof and combinations of other biodegradable polymers. Other polymers that may be used include polyesters, polypropylene, polyethylene and polycarbonate copolymers. Examples of suitable metals include, but are not limited to, stainless steel, titanium, tantalum, platinum, tungsten, gold and alloys of any of the above-mentioned metals. Examples of suitable alloys include platinum-iridium alloys, cobalt-chromium alloys including Elgiloy and Phynox, MP35N alloy and nickel-titanium alloys, for example, Nitinol.

The sheets forming the layers 12 of the multilayer rolled stent 10 may be made of shape memory materials such as superelastic Nitinol or spring steel, or may be made of materials which are plastically deformable. In the case of shape memory materials, the stent 10 may be provided with a memorized shape and then deformed to a reduced diameter shape. The stent 10 may restore itself to its memorized expanded rolled state upon being heated to a transition temperature and having any restraints removed therefrom.

The sheets forming the layers 12 of the multilayer rolled stent 10 may be created by methods including cutting or etching a design from a tubular stock, from a flat sheet which is cut or etched and which is subsequently rolled or from one or more interwoven wires or braids. Any other suitable technique which is known in the art or which is subsequently developed may also be used to manufacture the inventive stents 10 disclosed herein.

In some embodiments the multilayer rolled stent 10, may include one or more areas, bands, coatings, members, etc. that is (are) detectable by imaging modalities such as X-Ray, MRI, ultrasound, etc. In some embodiments at least a portion of the stent 10 and/or adjacent assembly is at least partially radiopaque.

In some embodiments at least a portion of the multilayer rolled stent 10 is configured to include one or more mechanisms for the delivery of a therapeutic agent. Often the agent will be in the form of a coating or other layer (or layers) of material placed on a surface region of the multilayer rolled stent 10, which is adapted to be released at the site of the stent's implantation or areas adjacent thereto. In one embodiment, each layer 12 of the multilayer rolled stent 10 delivers a different therapeutic agent. In one embodiment, the outer layer 12 (i.e. the layer in contact with the vessel wall) delivers a different therapeutic agent than the inner layer 12 (i.e. the layer 12 that defines the flow path of the stent 10). In one embodiment, the outer surface of the outer layer 12 is microporous to enhance vessel ingrowth into the multilayer rolled stent 10 so that the attachment of the multilayer rolled stent 10 to the vessel is enhanced and thrombogenicity is improved while the other layers 12 of the multilayer rolled stent 10 deliver at least one therapeutic agent. In this application, microporous means that the outer surface has perforations with diameters of about 0.001 inches (0.0254 mm) or less.

A therapeutic agent may be a drug or other pharmaceutical product such as non-genetic agents, genetic agents, cellular material, etc. Some examples of suitable non-genetic therapeutic agents include but axe not limited to: anti-thrombogenic agents such as heparin, heparin derivatives, vascular cell growth promoters, growth factor inhibitors, Paclitaxel, etc. Where an agent includes a genetic therapeutic agent, such a genetic agent may include but is not limited to: DNA, RNA and their respective derivatives and/or components; hedgehog proteins, etc. Where a therapeutic agent includes cellular material, the cellular material may include but is not limited to: cells of human origin and/or non-human origin as well as their respective components and/or derivatives thereof. Where the therapeutic agent includes a polymer agent, the polymer agent may be a polystyrene-polyisobutylene-polystyrene triblock copolymer (SIBS), polyethylene oxide, silicone rubber and/or any other suitable substrate.

## Claims

1. A rolled sheet multilayer stent (10), the rolled sheet multilayer stent (10) having an unrolled state, a rolled state, an outer surface layer (12a), and an inner surface layer (12b), the rolled sheet multilayer stent comprising a first sheet and a second sheet, the first and second sheets each having a first edge (18a, 18b), a second edge (20a, 20b) and at least one cell design, the at least one cell design comprising a plurality of struts (32, 34) forming a plurality of cells (40) **characterised in that**, in the unrolled state the first and second sheets lay on top of one another so that the first edge (18a) of the first sheet and the first edge (18b) of the second sheet form a first edge of the rolled sheet multilayer stent, in the rolled state the first edge (18a) of the first sheet opposite from the second edge (20a) of the first sheet and the first edge (18b) of the second sheet opposite from the second edge (20b) of the second sheet, the first sheet having a first circumferential length, the second sheet having a second circumferential length, the first circumferential length greater than the second circumferential length.

2. The stent (10) of claim 1, the rolled sheet multilayer stent (10) having a proximal end (14) and a distal end (16), the first sheet forming a substantial portion of the outer surface layer of the rolled sheet multilayer stent (10), the second sheet forming a substantial portion of the inner surface layer, the first sheet and the second sheet each having a proximal end and a distal end, the proximal end of the first sheet forming the proximal end of the rolled sheet multilayer stent, the distal end of the second sheet forming the distal end of the rolled sheet multilayer stent, the distal end of the first sheet proximal to the distal end of the second sheet and the proximal end of the second sheet distal to the proximal end of the first sheet.

3. The stent (10) of claim 1, the at least one cell design of the first and the second sheets selected from at least one member of the group consisting of closed cell (40), open cell, free- form cell and any combination thereof.

4. The stent (10) of claim 3, the first and second sheets each having a cell design, the cell design of the first sheet different from the cell design of the second sheet.

5. The stent (10) of claim 4, the cell design of the first sheet an open cell design and the cell design of the second sheet a closed cell design.

6. The stent (10) of claim 3, the first and second sheets having the same cell design.

7. The stent of claim 3, the rolled sheet multilayer stent having a cell design comprising a plurality of cells, the plurality of cells of the rolled sheet multilayer stent smaller than the plurality of cells of either the first sheet or the second sheet.

8. The stent of claim 3, the first and second sheet each having two cell designs, a first cell design in a first section of the sheet, a second cell design in a second section of the sheet, the first section and the second section of the sheet separated by a circumferential strut.

9. The stent of claim 8, the first cell design of the first sheet an open cell design, the second cell design of the first sheet a closed cell design, the first cell design of the second sheet a closed cell design, and the second cell design of the second sheet an open cell design.

10. The stent of claim 9, the first cell design of the first sheet the same as the second cell design of the second sheet and the second cell design of the first sheet the same as the first cell design of the first sheet.

11. The stent of claim 1, at least one of the first sheet and the second sheet delivering a therapeutic agent selected from at least one member of the group consisting of a non-genetic therapeutic agent, a genetic therapeutic agent, cellular material, a polymer agent, and any combination thereof.

12. The stent of claim 11, the first sheet and the second sheet delivering different therapeutic agents.

13. The stent of claim 1, the outer surface layer of the rolled sheet multilayer stent being microporous.

## Patentansprüche

1. Ein gerollter flächenförmiger Mehrschichtenstent (10), der einen ungerollten Zustand, einen gerollten Zustand, eine äußere Oberflächenschicht (12a) und eine innere Oberflächenschicht (12b) aufweist, der gerollte flächenförmige Mehrschichtenstent umfasst eine erste Lage und ein zweite Lage, wobei die erste und die zweite Lage jeweils eine erste Kante (18a, 18b), eine zweite Kante (20a, 20b) und mindestens ein Zelldesign aufweisen, wobei das mindestens eine Zelldesign eine Vielzahl an Streben (32, 34), die eine Vielzahl an Zellen (40) formen, aufweist, **dadurch gekennzeichnet, dass** in dem ungerollten Zustand die erste und die zweite Lage aufeinander liegen, so dass die erste Kante (18a) der ersten Lage und die erste Kante (18b) der zweiten Lage eine erste Kante des gerollten flächenförmigen Mehrschichtenstents bilden, in dem gerollten Zustand die erste Kante (18a) der ersten Lage gegenüber der zweiten Kante (20a) der ersten Lage und die erste Kante (18b) der zweiten Lage gegenüber der zweiten Kante (20b) der zweiten Lage liegen, wobei die erste Lage eine Umfangslänge aufweist, die zweite Lage eine Umfangslänge aufweist und die erste Umfangslänge größer als die zweite Umfangslänge ist.

2. Der Stent (10) nach Anspruch 1, wobei der gerollte flächenförmige Mehrschichtenstent (10) ein proximales Ende (14) und ein distales Ende (16) aufweist, die erste Lage einen wesentlichen Teil der äußeren Oberflächenschicht des gerollten flächenförmigen Mehrschichtenstent (10) bildet, die zweite Lage einen wesentlichen Teil der inneren Oberflächenschicht bildet, die erste Lage und die zweite Lage jeweils ein proximales und ein distales Ende aufweisen, wobei das proximale Ende der ersten Lage das proximale Ende des gerollten flächenförmigen Mehrschichtenstents bildet, das distale Ende der zweiten Lage das distale Ende des gerollten flächenförmigen Mehrschichtenstents bildet und das distale Ende der ersten Lage proximal zu dem distalen Ende der zweiten Lage und das proximale Ende der zweiten Lage distal zu dem proximalen Ende der ersten Lage ist.

3. Der Stent (10) nach Anspruch 1, wobei das mindestens eine Zelldesign der ersten und der zweiten Lage ausgewählt von mindestens einem Mitglied der Gruppe bestehend aus geschlossenen Zellen (40), offene Zellen, frei geformten Zellen und irgendeine Kombination davon sind.

4. Der Stent (10) nach Anspruch 3, bei dem die erste und die zweite Lage jeweils ein Zelldesign aufweisen, wobei das Zelldesign der ersten Lage verschieden von dem Zelldesign der zweiten Lage ist.

5. Der Stent (10) nach Anspruch 4, wobei das Zelldesign der ersten Lage ein offenes Zelldesign ist und das Zelldesign der zweiten Lage ein geschlossenes Zelldesign ist.

6. Der Stent (10) nach Anspruch 3, wobei die erste und die zweite Lage das gleiche Zelldesign haben.

7. Der Stent (10) nach Anspruch 3, bei dem der gerollte flächenförmige Mehrschichtenstent ein Zelldesign hat, das eine Vielzahl an Zellen umfasst, wobei die Vielzahl an Zellen des gerollten flächenförmigen Mehrschichtenstents kleiner als die Vielzahl der Zellen der entweder ersten Lage oder der zweiten Lage ist.

8. Der Stent (10) nach Anspruch 3, bei dem die erste und die zweite Lage jeweils zwei Zelldesigne aufweisen, ein erstes Zelldesign in einem ersten Abschnitt und ein zweites Zelldesign in einem zweiten Abschnitt der Lage, wobei der erste Abschnitt und der zweite Abschnitt der Lage durch eine Umfangsstrebe getrennt sind

9. Der Stent (10) nach Anspruch 8, bei dem das erste Zelldesign der ersten Lage ein offenes Zelldesign ist, das zweite Zelldesign der ersten Lage ein geschlossenes Zelldesign ist, das erste Zelldesign der zweiten Lage ein geschlossenes Zelldesign ist und das zweite Zelldesign der zweiten Lage ein offenes Zelldesign ist.

10. Der Stent (10) nach Anspruch 9, bei dem das erste Zelldesign der ersten Lage das gleiche wie das zweite Zelldesign der zweiten Lage und das zweite Zelldesign der ersten Lage das gleiche wie das erste Zelldesign des ersten Lage ist.

11. Der Stent (10) nach Anspruch 1, wobei mindestens eine der ersten Lage und der zweiten Lage ein Therapeutikum ausgewählt aus mindestens einem Mitglied der Gruppe bestehend aus Nicht-Gentherapeutika, Gentherapeutika, zellulärem Material, einem Polymermittel und irgendeine Kombination davon abgibt.

12. Der Stent (10) nach Anspruch 11, wobei die erste Lage und die zweite Lage unterschiedliche Therapeutika abgeben.

13. Der Stent (10) nach Anspruch 1, wobei die äußere Oberflächenschicht des gerollten flächenförmiger Mehrschichtenstents mikroporös ist.

## Revendications

1. Stent (10) multicouche à feuille enroulée, le stent (10) multicouche à feuille enroulée ayant un état non enroulé, un état enroulé, une couche superficielle extérieure (12a), et une couche superficielle intérieure (12b), le stent multicouche à feuille enroulée comprenant une première feuille et une deuxième feuille, les première et deuxième feuilles ayant chacune un premier bord (18a, 18b), un deuxième bord (20a, 20b) et au moins un modèle de cellule, le modèle de cellule au moins au nombre de un comprenant une pluralité de montants (32, 34) formant une pluralité de cellules (40), **caractérisé en ce que**, dans l'état non enroulé, les première et deuxième feuilles sont placées l'une sur l'autre de sorte que le premier bord (18a) de la première feuille et le premier bord (18b) de la deuxième feuille forment un premier bord du stent multicouche à feuille enroulée, dans l'état enroulé le premier bord (18a) de la deuxième feuille en face du deuxième bord (20a) de la première feuille et le premier bord (18b) de la deuxième feuille en face du deuxième bord (20b) de la deuxième feuille, la première feuille ayant une première longueur circonférentielle, la deuxième feuille ayant une deuxième longueur circonférentielle, la première longueur circonférentielle étant plus grande que la deuxième longueur circonférentielle.

2. Stent (10) selon la revendication 1, le stent (10) multicouche à feuille enroulée ayant une extrémité proximale (14) et une extrémité distale (16), la première feuille formant une portion substantielle de la couche superficielle extérieure du stent (10) multicouche à feuille enroulée, la deuxième feuille formant une portion substantielle de la couche superficielle intérieure, la première feuille et la deuxième feuille ayant chacune une extrémité proximale et une extrémité distale, l'extrémité proximale de la première feuille formant l'extrémité proximale du stent multicouche à feuille enroulée, l'extrémité distale de la deuxième feuille formant l'extrémité distale du stent multicouche à feuille enroulée, l'extrémité distale de la première feuille proximale à l'extrémité distale de la deuxième feuille et l'extrémité proximale de la deuxième feuille distale à l'extrémité proximale de la première feuille.

3. Stent (10) selon la revendication 1, le modèle de cellule au moins au nombre de un des première et deuxième feuilles sélectionnés parmi au moins un groupe constitué d'une cellule fermée (40), d'une cellule ouverte, d'une cellule de forme libre et de n'importe quelle combinaison de celles-ci.

4. Stent (10) selon la revendication 3, les première et deuxième feuilles ayant chacune un modèle de cellule, le modèle de cellule de la première feuille étant différent du modèle de cellule de la deuxième feuille.

5. Stent (10) selon la revendication 4, le modèle de cellule de la première feuille étant un modèle de cellule ouvert et le modèle de cellule de la deuxième feuille étant un modèle de cellule fermé.

6. Stent (10) selon la revendication 3, les première et deuxième feuilles ayant le même modèle de cellule.

7. Stent selon la revendication 3, le stent multicouche à feuille enroulée ayant un modèle de cellule comprenant une pluralité de cellules, la pluralité de cellules du stent multicouche à feuille enroulée étant plus petite que la pluralité de cellules soit de la première feuille soit de la deuxième feuille.

8. Stent selon la revendication 3, la première et deuxième feuille ayant chacune deux modèles de cellule, un premier modèle de cellule dans une première section de la feuille, un deuxième modèle de cellule dans une deuxième section de la feuille, la première section et la deuxième section de la feuille étant séparées par un montant circonférentiel.

9. Stent selon la revendication 8, le premier modèle de cellule de la première feuille étant un modèle de cellule ouvert, le deuxième modèle de cellule de la première feuille un modèle de cellule fermé, le premier modèle de cellule de la deuxième feuille un modèle de cellule fermé, et le deuxième modèle de cellule de la deuxième feuille un modèle de cellule ouvert.

10. Stent selon la revendication 9, le premier modèle de cellule de la première feuille étant le même que le deuxième modèle de cellule de la deuxième feuille et le deuxième modèle de cellule de la première feuille le même que le premier modèle de cellule de la première feuille.

11. Stent selon la revendication 1, au moins une parmi la première feuille et la deuxième feuille délivrant un agent thérapeutique sélectionné parmi au moins un membre du groupe constitué d'un agent thérapeutique non génétique, d'un agent thérapeutique génétique, d'un matériau cellulaire, d'un agent polymère et de n'importe quelle combinaison de ceux-ci.

12. Stent selon la revendication 11, la première feuille et la deuxième feuille délivrant différents agents thérapeutiques.

13. Stent selon la revendication 1, la couche superficielle extérieure du stent multicouche à feuille enroulée étant microporeuse.
